# EUROPEAN PATENT SPECIFICATION

(11) **EP 2 084 165 B1**
(45) Date of publication and mention of the grant of the patent: **12.06.2013**
(21) Application number: 06809572.8
(22) Date of filing: 11.10.2006
(51) Int. Cl.: C07D 513/04, C07D 417/12, C07D 279/16

(54) **POSITIVELY CHARGED WATER-SOLUBLE PRODRUGS OF OXICAMS AND RELATED COMPOUNDS WITH VERY HIGH SKIN PENETRATION RATE**
POSITIV GELADENE, WASSERLÖSLICHE PRODRUGS VON OXICAMEN UND VERWANDTEN VERBINDUNGEN MIT SEHR HOHER HAUTPENETRATIONSRATE
PRO-MÉDICAMENTS D'OXICAMS SOLUBLES DANS L'EAU ET CHARGÉS POSITIVEMENT ET COMPOSÉS APPARENTÉS AVEC UNE VITESSE DE PÉNÉTRATION DE LA PEAU TRÈS ÉLEVÉE

(43) Date of publication of application: 05.08.2009
(73) Proprietor: Techfields Biochem Co. Ltd, Shanghai N/A 200444 (CN); Yu, Chongxi, Plainfield, Illinois 60585 (US)
(72) Inventor: YU, Chongxi, Plainfield, Illinois 60585 (US); XU, Lina, Shanghai N/A 200444 (CN)
(74) Representative: Viering, Jentschura & Partner
(86) International application number: PCT/IB2006/053741
(87) International publication number: WO 2008/044095

(56) References cited:
- EP-A2- 0 208 404
- WO-A1-90/02141
- US-A- 4 551 452
- US-A- 5 081 118
- US-A- 5 607 691
- US-A1- 2004 254 182
- VENUTI M C ET AL: "SYNTHESIS AND BIOLOGICAL EVALUATION OF OMEGA-(N,N,N-TRIALKYLAMMONIUM) ALKYL ESTERS AND THIOESTERS OF CARBOXYLIC ACID NONSTEROIDAL ANTIINFLAMMATORY AGENTS", PHARMACEUTICAL RESEARCH, KLUWER ACADEMIC PUBLISHERS, NEW YORK, NY, US, vol. 6, no. 10, 1 January 1989 (1989-01-01), pages 867-873, XP001098334, ISSN: 0724-8741, DOI: DOI:10.1023/A:1015960522189
- NICOLAS COLETTE ET AL: "New Quaternary Ammonium Oxicam Derivatives Targeted toward Cartilage: Synthesis, Pharmacokinetic Studies, and Antiinflammatory Potency", JOURNAL OF MEDICINAL CHEMISTRY, AMERICAN CHEMICAL SOCIETY, WASHINGTON, US, vol. 42, 1 January 1999 (1999-01-01), pages 5235-5240, XP002165071, ISSN: 0022-2623, DOI: DOI:10.1021/JM991120O
- GIRAUD I ET AL: "Application to a Cartilage Targeting Strategy: Synthesis and in Vivo Biodistribution of 14C-Labeled Quaternary Ammonium-Glucosamine Conjugates", BIOCONJUGATE CHEM., vol. 11, 17 February 2000 (2000-02-17), pages 212-218, XP002611626,
- SONG N ET AL: "Synthesis of a derivative quaternary ammonium-ibuprofen", JOURNAL OF OCEAN UNIVERSITY OF QINGDAO,, vol. 32, no. 6, 1 January 2002 (2002-01-01), pages 911-913, XP001539131,

## Description

### Technical Fields

The present invention relates to positively charged and watersoluble pro-drugs of oxicams and related compounds their preparations, and their medicinal use in treating any oxicams-treatable conditions in humans or animals. More specifically, the present invention is to overcome the side effects that are associated with the use of oxicams and related compounds. These pro-drugs can be administered orally or transdermally.

### Background Art

Piroxicam, sudoxiam, lomoxicam, tenoxicam, ampiroxicam, lomoxicam, isoxicam, cinnoxicam, meloxicam, and related compounds are members of enolic acid class of 4-hydroxy-1,2-benzothiazine carboxamides with anti-inflammatory and analgesic properties. The first member of this class, piroxicam, was introduced in the United States in 1982 as Feldene(Pfizer). Oxicams are the leading analgesic and antipyretic drugs. They are used to relieve symptoms of rheumatoid arthritis, osteoarthritis and for fever. Other agents of oxicams are disclosed in U.S. Pat. Nos. 3,787,324, 3,822,258, 4,180,662 and 4,376,768.

Unfortunately, a number of side effects are associated with the use of oxicams and related compounds, most notably GI disturbances such as dyspepsia, gastroduodenal bleeding, gastric ulcerations, and gastritis. Fishman (Fishman; Robert, U.S. Pat. No. 7,052,715) indicated that an additional problem associated with oral medications, is that the concentration levels achieved in the bloodstream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Roentsch, et al., U.S. Pat. No. 5,654,337, Park, et al., U.S. Pat. No. 6,190,690, Pearson et al. U.S. Pat. No. 6,528,040, and Botknecht et al. U.S. Pat. No. 5,885,597) have tried to develop a delivery system for transdermal application by formulation. It is very difficult, however, to deliver therapeutically effective plasma levels of these kind drugs into the host by formulation, due to the slow skin penetration rate. Susan Milosovich, et al. designed and prepared testosteronyl-4-dimethylaminobutyrate.HCl (TSBH), which has a lipophilic portion and a tertiary amine groups that exists in the protonated form at physiological pH. They found that the prodrug diffuses through human skin ~60 times faster than does the drug itself [Susan Milosovich, et al., J. Pharm. Sci., 82, 227(1993).

EP 0208404 A2 discloses benzothiazine dioxide derivatives including substituted piroxicam derivatives and generally teaches that oxicam prodrugs have good bioavailability, are soluble, stable and highly effective as anti-inflammatory/analgesic drugs.

US 5,607,691 discloses piroxicam derivatives and further explains that modifying a drug by a charged group can improve transport of the drug through membranes.

### Disclosure of Invention

### Technical Problem

Piroxicam, sudoxiam, lornoxicam, tenoxicam, ampiroxicam, lomoxicam, isoxicam, cinnoxicam, meloxicam, and related compounds are used for the relief of signs and symptoms of rheumatoid arthritis and osteoarthritis and for relief of fever.

Unfortunately, a number of side effects are associated with the use of oxicams and related compounds, most notably GI disturbances such as dyspepsia, gastroduodenal bleeding, gastric ulcerations, and gastritis.

### Technical Solution

This invention relates positively charged pro-drugs of oxicams and related compounds their preparation, and their medicinal use. The pro-drugs of oxicams and related compounds have the general formula (1) 'Structure 1'. 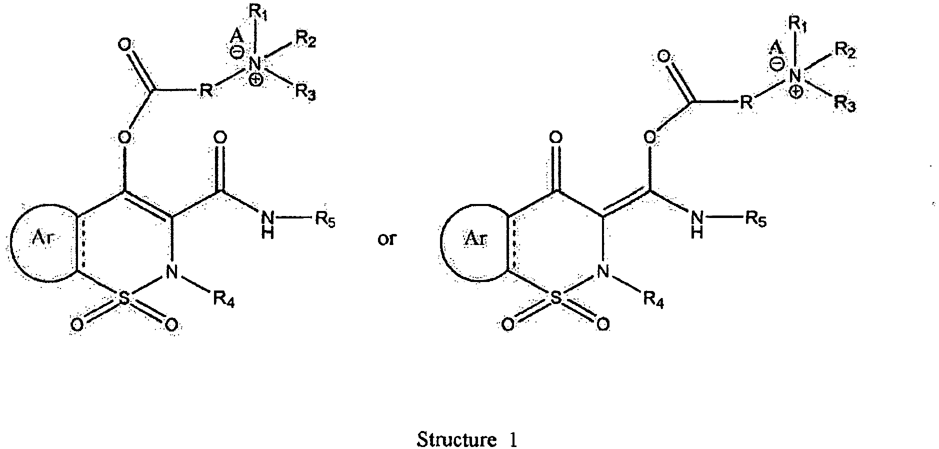 Wherein, R represents a branched or straight chain, -(CH₂)ₙ-, wherein n=0,1, 2, 3,4, 5, 6, 7, 8, 9, 10, ; R₁ represents H, one of any alkyl, alkyloxyl, alkenyl or alkynyl residues having up to 12 carbon atoms, aryl or heteroaryl residues; R₂ represents H, one of any alkyl, alkyloxy, alkenyl or alkynyl residues having up to 12 carbon atoms, aryl or heteroaryl residues; R₃ represents H ; R₄ presents H, CH₃, C₂H₅, CF₃ or C₂F₅; A represents a negative ion such as Cl⁻, Br⁻, F⁻, I⁻, AcO⁻, citrate; R₅ represents aryl or heteroaryl system, selected from: 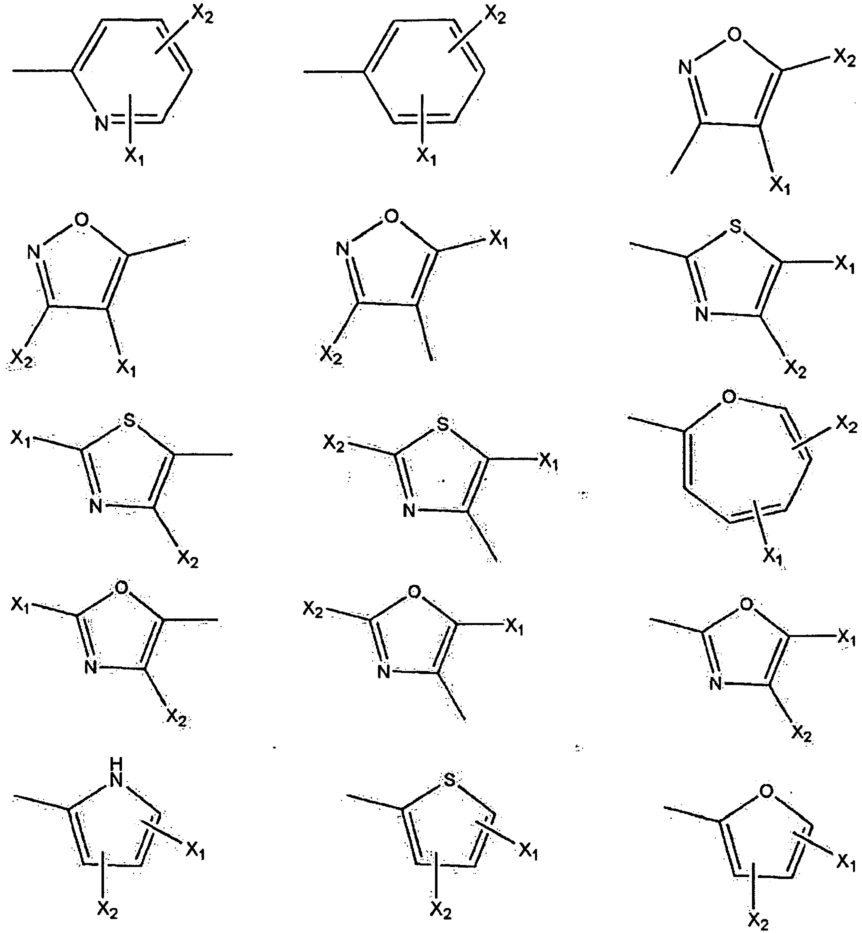 Wherein, X₁ and X₂ represent H, F, Cl, Br, I, CF₃, C₂F₅, SO₂CF₃, SO₂CH₃, NO₂, CN, alkyl, alkyloxyl, alkenyl or alkynyl residues having up to 8 carbon atoms. 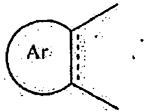 represents aryl or heteroaryl system, selected from: 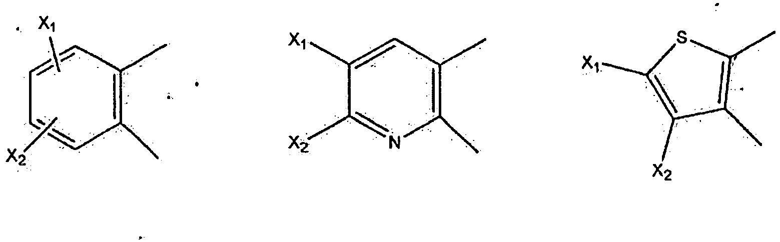 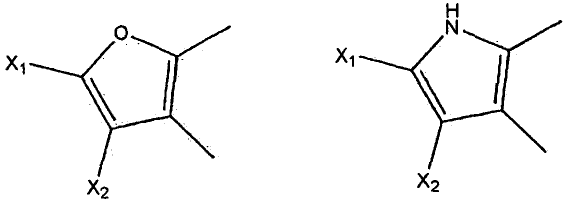 Wherein, X₁ and X₂ represent H, F, Cl, Br, I, CF₃, C₂F₅, SO₂CF₃, SO₂CH₃, NO₂, alkyl, alkyloxyl, alkenyl or alkynyl residues having up to 8 carbon atoms. .

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane and into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

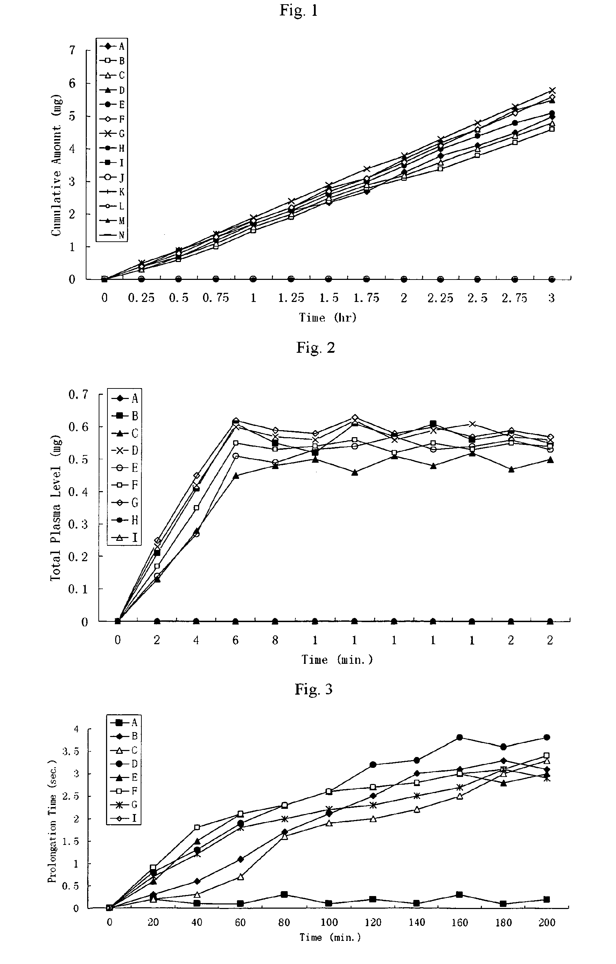

The goal of this invention is to avoid the side effects of oxicams and related compounds by increasing their solubility in gastric juice and moisture of skin and their penetration rate through the membrane and skin barrier which will make them administrable transdermally (topical application). These novel pro-drugs have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubility of
4-N,N-dimethylaminobutyryloxy-2-methyl-N-2-pyridinyl-2H,1,2-benzothiazine-3-carb oxamide 1,1-dioxide.HCl, N-(2-thiazolyl)-4-N,N-dimethylaminobutyryloxy-2-methyl-2H,1,2-benzothiazine-3-carbo xamide 1,1-dioxide.HCl,
6-chloro-4-N,N-dimethylaminobutyryloxy-2-methyl-N-2-pyridinyl-2H-thieno[2,3-e]-1 ,2-thiazine-3-carboxamide 1,1-dioxide.HCl,
4-N,N-dimethylaminobutyryloxy-2-methyl-N-2-pyridinyl-2H-thieno[2,3-e]-1,2-thiazin e-3-carboxamide 1,1-dioxide.HCl,
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl, 4-N,N-dimethylaminobutyryloxy -
2-methyl-N-[5-Methyl-3-isoxolyl-2H-1,2-benzothiazine-3-carboxamide 1,1-dioxide] .HCl, 4- N,N-dimethylaminobutyryloxy -2-methyl-N-(5-methyl-2-thiazolyl)-2H-1,2-benzothiazine-3-carboxamide-1,1-dioxide.HCl, Piroxicam, sudoxiam, lornoxicam, tenoxicam, lomoxicam, isoxicam, and meloxicam in water are >300 mg, >300 mg, >300 mg, >300 mg, >300 mg, >300 mg, >300 mg, <0.1 mg, <0.1 mg, <0.1 mg, <0.1 mg, <0.1 mg, <0.1 mg, and <0.1 mg/ml, In many instances, the lowest or rate-limiting step in the sequence is the dissolution of the drug. Oxicams and related compounds have a very low solubility in gastric juice or the moisture of skin. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. The pH of the stomach is 1-3, so the negative charge on the phosphate head group of the membrane of the gastric mucosa is bonded with proton (H⁺). The positive charges of these prodrugs cannot bond to phosphate head group of the gastric mucosa. Thus, prodrugs will not damage the stomach. The penetration rates of
4-N,N-dimethylaminobutyryloxy-2-methyl-N-2-pyridinyl-2H,1,2-benzothiazine-3-carb oxamide 1,1-dioxide.HCl, N-(2-thiazoyl)-4-N,N-dimethylaminobutyryloxy-2-methyl-2H,1,2-benzothiazine-3-carbo xamide 1,1-dioxide.HCl,
6-chloro-4-N,N-dimethylaminobutyryloxy-2-methyl-N-2-pyridinyl-2H-thieno[2,3-e]-1 ,2-thiazine-3-carboxamide 1,1-dioxide.HCl,
4-N,N-dimethylaminobutyryloxy-2-methyl-N-2-pyridinyl-2H-thieno[2,3-e]-1,2-thiazin e-3-carboxamide 1,1-dioxide.HCl,
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl, 4-N,N-dimethylaminobutyryloxy -2-methyl-N -
[5-Methyl-3-isoxolyl-2H-1,2-benzothiazine-3-carboxamide 1,1-dioxide].HCl, 4-N,N-dimethylaminobutyryloxy -2-methyl-N-(5-methyl-2-thiazolyl)-2H-1,2-benzothiazine-3-carboxamide-1,1-dioxide.HCl, Piroxicam, sudoxiam, lornoxicam, tenoxicam, lomoxicam, isoxicam, meloxicam, and related compounds through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 20% solution of
4-N,N-dimethylaminobutyryloxy-2-methyl-N-2-pyridinyl-2H,1,2-benzothiazine-3-carb oxamide 1,1-dioxide.HCl, N-(2-thiazolyl)-4-N,N-dimethylaminobutyryloxy-2-methyl-2H,1,2-benzothiazine-3-carbo xamide 1,1-dioxide.HCl,
6-chloro-4-N,N-dimethylaminobutyryloxy-2-methyl-N-2-pyridinyl-2H-thieno[2,3-e]-1 ,2-thiazine-3-carboxamide 1,1-dioxide.HCl,
4-N,N-dimethylaminobutyryloxy-2-methyl-N-2-pyridinyl-2H-thieno[2,3-e]-1,2-thiazin e-3-carboxamide 1,1-dioxide.HCl,
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl, 4-N,N-dimethylaminobutyryloxy -
2-methyl-N-[5-Methyl-3-isoxolyl-2H-1,2-benzothiazine-3-carboxamide 1,1-dioxide] .HCl, and 4- N,N-dimethylaminobutyryloxy -2-methyl-N-(5-methyl-2-thiazolyl)-2H-1,2-benzothiazine-3-carboxamide-1,1-dioxide.HCl or a 20% suspension of Piroxicam, sudoxiam, lornoxicam, tenoxicam, lomoxicam, isoxicam, and meloxicam in 2mL of pH 7.4 phosphate buffer (0.2M) are shown in Figure 1. Apparent flux values of 1.7 mg, 1.5 mg, 1.6 mg, 1.8 mg, 1.7 mg, 1.8 mg, 1.9 mg, 0.001 mg, 0.001 mg, 0.001 mg, 0.001 mg, 0.001 mg, 0.001 mg, and 0.001 mg/cm²/h were calculated for
4-N,N-dimethylaminobutyryloxy-2-methyl-N-2-pyridinyl-2H,1,2-benzothiazine-3-carb oxamide 1,1-dioxide.HCl, N-(2-thiazoyl)-4-N,N-dimethylaminobutyryloxy-2-methyl-2H,1,2-benzothiazine-3-carbo xamide 1,1-dioxide.HCl,
6-chloro-4-N,N-dimethylaminobutyryloxy-2-methyl-N-2-pyridinyl-2H-thieno[2,3-e]-1 ,2-thiazine-3-carboxamide 1,1-dioxide.HCl,
4-N,N-dimethylaminobutyryloxy-2-methyl-N-2-pyridinyl-2H-thieno[2,3-e]-1,2-thiazin e-3-carboxamide 1,1-dioxide.HCl,
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl, 4-N,N-dimethylaminobutyryloxy -2-methyl-N - [5-Methyl-3-isoxolyl-2H-1,2-benzothiazine-3-carboxamide 1,1-dioxide].HCl, and 4-N,N-dimethylaminobutyryloxy-2-methyl-N-(5-methyl-2-thiazolyl)-2H-1,2-benzothiazine-3-carboxamide-1,1-dioxide.HCl , Piroxicam, sudoxiam, lornoxicam, tenoxicam, lomoxicam, isoxicam, and meloxicam. The results suggest that the positive charge on the dialkyaminoethyl group has a very important role in the passage of the drug across the membrane or skin barrier. Other prodrugs of the general 'Structure 1' have very high penetration rates and are very close to that of 4-N,N-dimethylaminobutyryloxy-2-methyl-N-2-pyridinyl-2H,1,2-benzothiazine-3-carb oxamide 1,1-dioxide.HCl.

The in vivo rates of penetration of
4-N,N-dimethylaminobutyryloxy-2-methyl-N-2-pyridinyl-2H,1,2-benzothiazine-3-carb oxamide 1,1-dioxide.HCl, N-(2-thiazolyl)-4-N,N-dimethylaminobutyryloxy-2-methyl-2H,1,2-benzothiazine-3-carbo xamide 1,1-dioxide.HCl,
6-chloro-4-N,N-dimethylaminobutyryloxy-2-methyl-N-2-pyridinyl-2H-thieno[2,3-e]-1 ,2-thiazine-3-carboxamide 1,1-dioxide.HCl,
4-N,N-dimethylaminobutyryloxy-2-methyl-N-2-pyridinyl-2H-thieno[2,3-e]-1,2-thiazin e-3-carboxamide 1,1-dioxide.HCl,
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl, 4-N,N-dimethylaminobutyryloxy - 2-methyl-N-[5-Methyl-3-isoxolyl-2H-1,2-benzothiazine-3-carboxamide 1,1-dioxide] .HCl, 4- N,N-dimethylaminobutyryloxy -2-methyl-N-(5-methyl-2-thiazolyl)-2H-1,2-benzothiazine-3-carboxamide-1,1-dioxide.HCl, Piroxicam, sudoxiam, lornoxicam, tenoxicam, lomoxicam, isoxicam, and meloxicam through the skin of intact hairless mice were compared. The donor consisted of a 20% solution of these compounds in 1 mL of isopropanol applied to a 10 cm² on the backs of the hairless mice. Plasma levels of drugs were determined by a specific high-performance liquid chromatography method. The results (Figure 2) show that the peak levels of 4-N,N-dimethylaminobutyryloxy-2-methyl-N-2-pyridinyl-2H,1,2-benzothiazine-3-carb oxamide 1,1-dioxide.HCl, N-(2-thiazoyl)-4-N,N-dimethylaminobutyryloxy-2-methyl-2H,1,2-benzothiazine-3-carbo xamide 1,1-dioxide.HCl,
6-chloro-4-N,N-dimethylaminobutyryloxy-2-methyl-N-2-pyridinyl-2H-thieno[2,3-e]-1 ,2-thiazine-3-carboxamide 1,1-dioxide.HCl,
4-N,N-dimethylaminobutyryloxy-2-methyl-N-2-pyridinyl-2H-thieno[2,3-e]-1,2-thiazin e-3-carboxamide 1,1-dioxide.HCl,
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl, 4-N,N-dimethylaminobutyryloxy -
2-methyl-N-[5-Methyl-3-isoxolyl-2H-1,2-benzothiazine-3-carboxamide 1,1-dioxide] .HCl, 4- N,N-dimethylaminobutyryloxy -2-methyl-N-(5-methyl-2-thiazolyl)-2H-1,2-benzothiazine-3-carboxamide-1,1-dioxide.HCl were reached in -50 minutes after application of the donor systems.

It takes 1-2 hours for oxicams and related compounds to reach their peak plasma level when they are taken orally. The peak plasma levels were -0.005 mg/ml for piroxicam, and sudoxiam and -0.5 mg/ml for
4-N,N-dimethylaminobutyryloxy-2-methyl-N-2-pyridinyl-2H,1,2-benzothiazine-3-carb oxamide 1,1-dioxide.HCl, N-(2-thiazolyl)-4-N,N-dimethylaminobutyryloxy-2-methyl-2H,1,2-benzothiazine-3-carbo xamide 1,1-dioxide.HCl,
6-chloro-4-N,N-dimethylaminobutyryloxy-2-methyl-N-2-pyridinyl-2H-thieno[2,3-e]-1 ,2-thiazine-3-carboxamide 1,1-dioxide.HCl,
4-N,N-dimethylaminobutyryloxy-2-methyl-N-2-pyridinyl-2H-thieno[2,3-e]-1,2-thiazin e-3-carboxamide 1,1-dioxide.HCl,
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl, 4-N,N-dimethylaminobutyryloxy - 2-methyl-N-[5-Methyl-3-isoxolyl-2H-1,2-benzothiazine-3-carboxamide 1,1-dioxide] .HCl, 4- N,N-dimethylaminobutyryloxy-2-methyl-N-(5-methyl-2-thiazolyl)-2H-1,2-benzothiazine-3-carboxamide-1,1-dioxide.HCl (approximately 100 times difference). -0.5 mg/ml of acetaminophen and acetaminosalol in plasma is more than -50 times higher than plasma level for effective analgesia and effective antiinflammatory activity. This is a very exciting result. It will be very easy and fast to deliver therapeutically effective plasma level of oxicams into the host by administration of these prodrugs transdermally. These results suggest that the pro-drugs can be administered not only orally, but also transdermally for any kind of medical treatments. The in vivo rates of penetration of other pro-drugs of the general 'Structure 1' are close to that of
4-N,N-dimethylaminobutyryloxy-2-methyl-N-2-pyridinyl-2H,1,2-benzothiazine-3-carb oxamide 1,1-dioxide.HCl.

The acute toxicity of the prodrugs was investigated. The LD₅₀ orally in mice are: 550 mg/kg, 670 mg/kg, 580 mg/kg, 500 mg/kg, 610 mg/kg, 570 mg/kg, and 590 mg/kg, and 360 mg/kg for
4-N,N-dimethylaminobutyryloxy-2-methyl-N-2-pyridinyl-2H,1,2-benzothiazine-3-carb oxamide 1,1-dioxide.HCl, N-(2-thiazoyl)-4-N,N-dimethylaminobutyryloxy-2-methyl-2H,1,2-benzothiazine-3-carbo xamide 1,1-dioxide.HCl,
6-chloro-4-N,N-dimethylaminobutyryloxy-2-methyl-N-2-pyridinyl-2H-thieno[2,3-e]-1 ,2-thiazine-3-carboxamide 1,1-dioxide.HCl,
4-N,N-dimethylaminobutyryloxy-2-methyl-N-2-pyridinyl-2H-thieno[2,3-e]-1,2-thiazin e-3-carboxamide 1,1-dioxide.HCl,
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl, 4-N,N-dimethylaminobutyryloxy -2-methyl-N - [5-Methyl-3-isoxolyl-2H-1,2-benzothiazine-3-carboxamide 1,1-dioxide].HCl, 4-N,N-dimethylaminobutyryloxy-2-methyl-N-(5-methyl-2-thiazolyl)-2H-1,2-benzothiazine-3-carboxamide-1,1-dioxide.HCl and piroxicam. The prodrugs are less toxic than the parent drugs.

A good prodrug should go back to the parent drug in plasma. The prodrugs in this invention can be rapidly cleaved by the enzymes in human plasma in vitro. More than 90% of the pro-drugs are changed back to their parent drugs in a few minutes. Due to the pro-drugs having a much better absorption rate, the prodrugs will have more strength than their parent drugs at the same dosage. Oxicams demonstrated analgesic and antipyretic activity. The analgetic and antipyretic activities of these prodrugs were tested using piroxicam as a comparison.

Analgesic activity: The prolongation time of the pain threshold of a mouse tail was determined in accordance with the D'Amour-Smith Method (J. Pharmacol. Exp. Ther., 72, 74(1941)). After 20 mg/kg of these prodrugs were administered transdermally, the tails of mice were exposed to heat and the prolongation time of pain threshold was determined. The results obtained are shown in Figure 3. The prodrugs of oxicams have shown analgesic activity nicely when they were administered transdermally.

The number of writhings that occurred when mice were administered an acetic acid solution intraperitoneally were counted, and the rate of inhibition based on the control group was calculated.
4-N,N-dimethylaminobutyryloxy-2-methyl-N-2-pyridinyl-2H,1,2-benzothiazine-3-carb oxamide 1,1-dioxide.HCl (50 mg/kg, B), N-(2-thiazoyl)-4-N,N-dimethylaminobutyryloxy-2-methyl-2H,1,2-benzothiazine-3-carbo xamide 1,1-dioxide.HCl (50 mg/kg, C),
6-chloro-4-N,N-dimethylaminobutyryloxy-2-methyl-N-2-pyridinyl-2H-thieno[2,3-e]-1 ,2-thiazine-3-carboxamide 1,1-dioxide.HCl (50mg/kg, D),
4-N,N-dimethylaminobutyryloxy-2-methyl-N-2-pyridinyl-2H-thieno[2,3-e]-1,2-thiazin e-3-carboxamide 1,1-dioxide.HCl (50 mg/kg, E),
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl (50 mg/kg, F), 4- N,N-dimethylaminobutyryloxy - 2-methyl-N-[5-Methyl-3-isoxolyl-2H-1,2-benzothiazine-3-carboxamide 1,1-dioxide] .HCl (50 mg/kg, G), and 4- N,N-dimethylaminobutyryloxy -2-methyl-N-(5-methyl-2-thiazolyl)-2H-1,2-benzothiazine-3-carboxamide-1,1-dioxide.HCl (50 mg/kg, H) were administered transdermally the mice 60 minutes before the acetic acid solution was administered. The group A is the control group. The results are shown in Table 1.

**Table 1. The Rate of Writhings Inhibition by Prodrugs of Oxicams.**

| Compound | Dose (mg/kg) | No. of Writhings | Rate of Inhibition (%) |
|---|---|---|---|
| A | 0 | 35.0 | - |
| B | 50 | 15.6 | 55 |
| C | 50 | 15.7 | 55 |
| D | 50 | 16.5 | 53 |
| E | 50 | 16.9 | 53 |
| F | 50 | 17.5 | 50 |
| G | 50 | 15.8 | 55 |
| H | 50 | 18.2 | 48 |

The results show that the prodrugs demonstrate exceptional analgetic activity. Other compounds of the general 'Structure 1' show similar analgetic activity.

Antipyretic activity: Rats received a sterilized E. coli suspension as a pyrogen. The control group is group A. 2 hours later,
4-N,N-dimethylaminobutyryloxy-2-methyl-N-2-pyridinyl-2H,1,2-benzothiazine-3-carb oxamide 1,1-dioxide.HCl (25 mg/kg, B), N-(2-thiazoyl)-4-N,N-dimethylaminobutyryloxy-2-methyl-2H,1,2-benzothiazine-3-carbo xamide 1,1-dioxide.HCl (25 mg/kg, C),
6-chloro-4-N,N-dimethylaminobutyryloxy-2-methyl-N-2-pyridinyl-2H-thieno[2,3-e]-1 ,2-thiazine-3-carboxamide 1,1-dioxide.HCl (25mg/kg, D),
4-N,N-dimethylaminobutyryloxy-2-methyl-N-2-pyridinyl-2H-thieno[2,3-e]-1,2-thiazin e-3-carboxamide 1,1-dioxide.HCl (25 mg/kg, E),
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl (25 mg/kg, F), 4- N,N-dimethylaminobutyryloxy - 2-methyl-N-[5-Methyl-3-isoxolyl-2H-1,2-benzothiazine-3-carboxamide 1,1-dioxide] .HCl ( 25 mg/kg, G) , and 4- N,N-dimethylaminobutyryloxy -2-methyl-N-(5-methyl-2-thiazolyl)-2H-1,2-benzothiazine-3-carboxamide-1,1-dioxide.HCl (25 mg/kg, H) were administered transdermally. The body temperature of the rats was taken at 90 min. intervals before and after the administration of the test compounds. The results are shown in Table 2.

**Table 2. Antipyretic Activity of Prodrugs of Oxicams.**

| Compound | t = 0 min. | t = 90 min. | t = 180 min. | t = 270 min. |
|---|---|---|---|---|
| A (Control group) | 37.54±0.05 | 37.66±0.07 | 37.67±0.05 | 37.64±0.08 |
| B (25mg/kg) | 37.57±0.06 | 36.51±0.05 | 36.40±0.06 | 36.45±0.07 |
| C (25mg/kg) | 37.50±0.07 | 36.61±0.04 | 36.50±0.07 | 36.60±0.05 |
| D (25mg/kg) | 37.55±0.05 | 36.66±0.06 | 36.60±0.06 | 36.61±0.07 |
| E (25mg/kg) | 37.54±0.06 | 36.61±0.06 | 36.58±0.08 | 36.55±0.05 |
| F (25mg/kg) | 37.53±0.05 | 36.57±0.05 | 36.52±0.07 | 36.51±0.06 |
| G (25mg/kg) | 37.52±0.06 | 36.62±0.07 | 36.53±0.06 | 36.60±0.05 |
| H (25mg/kg) | 37.57±0.07 | 36.53±0.08 | 36.52±0.08 | 36.50±0.07 |

The results shown that the prodrugs demonstrated strong antipyretic activity at 25 mg/kg dose. Other compounds of the general 'Structure 1' show similar antipyretic activity.

It is also known that a high oral dose of some of NSAIAs shows an anti-reactive-antiasthmatic activity by inhibition of the cyclooxygenase activity. Due to their very high membrane penetration rate, these prodrugs can be used in treating asthma by spraying into the mouth or nose of the host.

These prodrugs can also be used to treat psoriasis, acne, sunburn or other skin disorders due to inhibition of the cyclooxygenase activity and very high skin penetration rate. They may useful for treating skin, lung, breast, and other cancers.

The present invention relates to pharmaceutical preparations comprising of prodrugs of the general 'Structure 1' in addition to customary auxiliaries and excipients, e.g. in the form of tablets, capsules or solutions for administration orally and in the form of solutions, lotion, ointment, emulsion or gel for transdermal administration. The new active compounds of the general 'Structure 1' can be combined with vitamins such as A, B, C or E or beta-carotene, or other pharmaceuticals, such as beta-carotene, Caffeine , pseudoephedrine, azapirone, folic acid, etc., for treating any oxicams-treatable conditions in humans or animals.

Transdermal therapeutic application systems of compounds of the general 'Structure 1' or a composition comprising of at least one compound of the general 'Structure 1', as an active ingredient, can be used for treating any oxicams-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables to reach constantly optimal therapeutic blood levels to increase effectiveness and reduce the side effects of oxicams and related compounds. These systems can be worn on the wrist, ankle, arm, leg, or any part of body.

The compounds of the general formula (1) 'Structure 1' indicated above can be prepared from oxicams and related compounds, by reaction with compounds of the general formula (2) 'Structure 2' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris(dimethylamino)phosphonium hexafluorophosphate, et al. 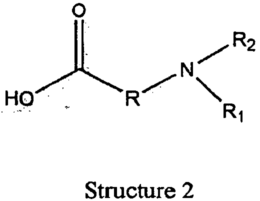 Wherein, R represents a branched or straight chain, -(CH₂)ₙ-, wherein n=0, 1, 2, 3, 4, 5, 6, 7, 8, 9, 10, ; R₁ represents H, one of any alkyl, alkyloxy, alkenyl, or alkynyl residues having up to 12 carbon atoms, aryl or heteroaryl residues; R₂ represents H, one of any alkyl, alkyloxy, alkenyl, or alkynyl residues having up to 12 carbon atoms, aryl or heteroaryl residues 9.

The compounds of the general formula (1) 'Structure 1' indicated above can be prepared from oxicams and related compounds, by reaction with compounds of the general formula (3) 'Structure 3'. 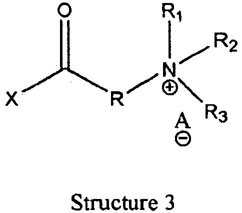 Wherein, R represents a branched or straight chain, -(CH₂)ₙ-, wherein n=0, 1, 2, 3, 4, 5, 6, 7, 8, 9, 10; R₁ represents H, one of any alkyl, alkyloxy, alkenyl, or alkynyl residues having up to 12 carbon atoms, aryl or heteroaryl residues; R represents H, one of any alkyl, alkyloxy, alkenyl, or alkynyl residues having up to 12 carbon atoms, aryl or heteroaryl residues; R represents H ; X represents halogen, or p-toluenesulphonyl, A⁻ represents negative ion such as Cl⁻, Br⁻, F⁻, I⁻, AcO⁻, citrate.

### Advantageous Effects

These pro-drugs of oxicams and related compounds have a lipophilic portion and a hydrophilic portion (the amine groups that exist in the protonated form at physiological pH). The positively charged amino groups of these pro-drugs have two major advantages. First, it largely increases the solubility of the drugs; when these new pro-drugs are administered orally in a dosage form such as a tablet, capsule, solution, or suspension, they will dissolve in gastric juice immediately. Second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, which is a semi-liquid concentrated aqueous solution or suspension. Experiment results show that more than 90% of the pro-drugs were changed back to the parent drugs in a few minutes. The pro-drugs have a much better absorption rate, and thus the pro-drugs. will have better strength than oxicams and related compounds at the same dosage. The experiment results suggest that the pro-drugs of oxicams diffuses through human skin ~100 times faster than do oxicams. It takes 1-2 hours for oxicams and related compounds to reach the peak plasma level when they are taken orally, but these pro-drugs only took about ~50 minutes to reach the peak plasma level. The most exciting result is that the pro-drugs can be administered not only orally, but also transdermally for any type of medical treatment and should avoid most of the side effects of oxicams and related compounds, most notably GI disturbances such as dyspepsia, gastroduodenal bleeding, gastric ulcerations, and gastritis. Another great benefit of the transdermal administration of these pro-drugs is that administering medication, especially to children, will be much easier.

### Description of Drawings

Figure 1: Cumulative amounts of
4-N,N-dimethylaminobutyryloxy-2-methyl-N-2-pyridinyl-2H,1,2-benzothiazine-3-carb oxamide 1,1-dioxide.HCl (A, 20% solution), N-(2-thiazolyl)-4-N,N-dimethylaminobutyryloxy-2-methyl-2H,1,2-benzothiazine-3-carbo xamide 1,1-dioxide.HCl (B, 20% solution),
6-chloro-4-N,N-dimethylaminobutyryloxy-2-methyl-N-2-pyridinyl-2H-thieno[2,3-e]-1 ,2-thiazine-3-carboxamide 1,1-dioxide.HCl (C, 20% solution), 4-N,N-dimethylaminobutyryloxy-2-methyl-N-2-pyridinyl-2H-thieno[2,3-e]-1,2-thiazin e-3-carboxamide 1,1-dioxide.HCl (D, 20% solution),
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl (E, 20% solution), 4- N,N-dimethylaminobutyryloxy -2-methyl-N -
[5-Methyl-3-isoxolyl-2H-1,2-benzothiazine-3-carboxamide 1,1-dioxide].HCl (F, 20% solution) , 4- N,N-dimethylaminobutyryloxy -2-methyl-N-(5-methyl-2-thiazolyl)-2H-1,2-benzothiazine-3-carboxamide-1,1-dioxide.HCl (G, 20% solution) , Piroxicam (H, 20% suspension), sudoxiam (I, 20% suspension), lomoxicam, (J, 20% suspension), tenoxicam (K, 20% suspension), lomoxicam (L, 20% suspension), isoxicam (M, 20% suspension), and meloxicam (N, 20% suspension) crossing isolated human skin tissue in Franz cells (n=5). In each case, the vehicle was pH 7.4 phosphate buffer (0.2 M).

Figure 2: Total plasma levels of drugs after topical application of 1 ml of
4-N,N-dimethylaminobutyryloxy-2-methyl-N-2-pyridinyl-2H,1,2-benzothiazine-3-carb oxamide 1,1-dioxide.HCl, N-(2-thiazolyl)-4-N,N-dimethylaminobutyryloxy-2-methyl-2H,1,2-benzothiazine-3-carbo xamide 1,1-dioxide.HCl,
6-chloro-4-N,N-dimethylaminobutyryloxy-2-methyl-N-2-pyridinyl-2H-thieno[2,3-e]-1 ,2-thiazine-3-carboxamide 1,1-dioxide.HCl,
4-N,N-dimethylaminobutyryloxy-2-methyl-N-2-pyridinyl-2H-thieno[2,3-e]-1,2-thiazin e-3-carboxamide 1,1-dioxide.HCl,
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl, 4-N,N-dimethylaminobutyryloxy -
2-methyl-N-[5-Methyl-3-isoxolyl-2H-1,2-benzothiazine-3-carboxamide 1,1-dioxide] .HCl, 4- N,N-dimethylaminobutyryloxy -2-methyl-N-(5-methyl-2-thiazolyl)-2H-1,2-benzothiazine-3-carboxamide-1,1-dioxide.HCl, Piroxicam, and sudoxiam in isopropanol to the backs of hairless mice (n=5).

Figure 3: The prolongation time of the pain threshold of mice tails after 20mg/kg of
4-N,N-dimethylaminobutyryloxy-2-methyl-N-2-pyridinyl-2H,1,2-benzothiazine-3-carb oxamide 1,1-dioxide.HCl (B), N-(2-thiazoyl)-4-N,N-dimethylaminobutyryloxy-2-methyl-2H,1,2-benzothiazine-3-carbo xamide 1,1-dioxide.HCl (C),
6-chloro-4-N,N-dimethylaminobutyryloxy-2-methyl-N-2-pyridinyl-2H-thieno[2,3-e]-1 ,2-thiazine-3-carboxamide 1,1-dioxide.HCl (D),
4-N,N-dimethylaminobutyryloxy-2-methyl-N-2-pyridinyl-2H-thieno[2,3-e]-1,2-thiazin e-3-carboxamide 1,1-dioxide.HCl (E),
8-chloro-(4-N,N-dimethylaminobutyryloxy-pyridine-2-ylamino-methylidene)-3-methy 1-2,2-dioxo-2λ⁶'7-dithia-3-azabicyclo[4,3,0]nona-8,10-dien-5-one.HCl (F), 4-N,N-dimethylaminobutyryloxy -
2-methyl-N-[5-Methyl-3-isoxolyl-2H-1,2-benzothiazine-3-carboxamide 1,1-dioxide] .HCl (G), and 4- N,N-dimethylaminobutyryloxy -2-methyl-N-(5-methyl-2-thiazolyl)-2H-1,2-benzothiazine-3-carboxamide-1,1-dioxide.HCl (H) were administered transdermally. Group A is the control group.

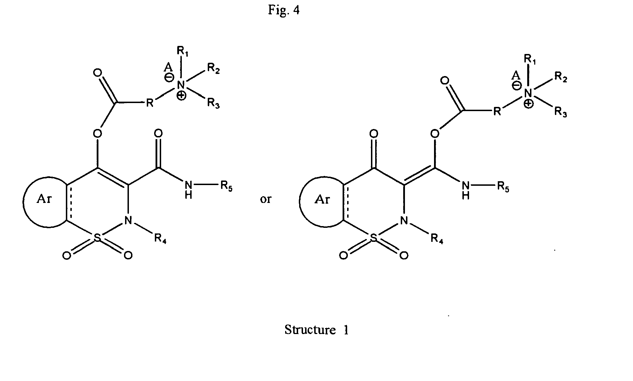

Figure 4: Structure 1. Wherein, R represents a branched or straight chain, -(CH₂)ₙ-, wherein n=0, 1, 2, 3, 4, 5, 6, 7, 8, 9, 10 ..., aryl residues or heteroaryl residues; R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R ₃ represents H, one of any alkyl, alkyloxy, alkenyl, or alkynyl residues having 1 to 12 carbon atoms, aryl or heteroaryl residues; R represents H, CH , C H , CF or C F ; R represents ary or heteroaryl system as indicated in the claim 1. Ar represents ary or heteroaryl rings as indicated in the claim 1. X represents halogen, or p-toluenesulphonyl, A⁻ represents Cl⁻, Br⁻, F, I⁻, AcO⁻, citrate, or any negative ions; and n=0, 1, 2, 3, 4, 5, 6, 7, 8, 9, 10....

### Best Mode

### Preparation of 4-N,N-dimethylaminobutyryloxy-2-methyl-N-2-pyridinyl-2H,1,2-benzothiazine-3-ca rboxamide 1,1-dioxide.HCl.

33.1 g (0.1 mol) of
4-hydroxy-2-methyl-N-2-pyridinyl-2H,1,2-benzothiazine-3-carboxamide 1,1-dioxide was dissolved in 200 ml of acetone and 250 ml of 10% NaHCO₃. 22.3 g (0.12 mol) of dimethylaminobutyryl chloride hydrochloride was added into the mixture. The mixture was stirred for 3 hours at RT. The solvents were evaporated off. 500 ml of ethyl acetate was added into the reaction mixture and the mixture was washed with 5% NaHCO₃ (1 x 200 ml) and water (3 x 100 ml). The organic solution was dried over anhydrous sodium sulfate. Sodium sulfate was removed by filtration. HCl gas (4 g) is bubbled into the solution. The solid product was collected by filtration. After drying, it yielded 40 g of the hygroscopic desired product (83.2%). Solubility in water: 250 mg/ml; Elementary analysis: C₂₁H₂₅ClN₄O₅S; MW: 480.96. Calculated % C: 52.44, H: 5.24, Cl: 7.37, N: 11.65, O: 16.63, S: 6.67; Found % C: 52.40, H: 5.27, Cl: 7.42, N: 11.60; O: 16.70, S: 6.61. ¹H-NMR (400 MHz, D₂O): δ: 2.00 (m, 2H), 2.23 (m, 2H), 2.46 (s, 3H), 2.85 (s, 6H), 3.18 (m, 2H), 6.60-6.70 (m, 2H), 7.20 (m, 1H), 7.40-7.44 (m, 2H), 7.56 (m, 1H), 7.80 (m, 1H), 8.10 (m, 1H).

### Mode for Invention

### Preparation of N-(2-thiazolyl)-4-N,N-dimethylaminobutyryloxy-2-methyl-2H,1,2-benzothiazine-3-carb oxamide 1,1-dioxsde.HCl

32.5g (0.1 mol) of N-(2-thiazolyl)-4-hydroxy-2-methyl-2H,1,2-benzothiazine-3-carboxamide 1,1-dioxide and 16 g (0.1 mol) of diethylaminobutyric acid were dissolved in 300 ml of dichloromethylene. The mixture is cooled to 0 °C with ice bath. 20.6 g (0.1 mol) of N, N'-Dicyclohexylcarbodiimide was added into the reaction mixture. The mixture was stirred for 1 hour at 0 °C and 2 hours at RT. The solid is removed by filtration. The dichloromethylene solution was washed with 5% NaHCO₃ (2 x 100 ml) and water (3 x 100 ml). The organic solution was dried over anhydrous sodium sulfate. Sodium sulfate was removed by filtration. HCl gas (4 g) is bubbled into the solution. The solid product was collected by filtration. After drying, it yielded 37 g of the hygroscopic desired product (76%). Solubility in water: 250 mg/ml; Elementary analysis: C₁₉H₂₃ ClN₄O₅S₂; MW: 486.99. Calculated % C: 46.86, H: 4.76, Cl: 7.28, N: 11.50, O: 16.43, S: 13.17; Found % C: 46.83, H: 4.78, Cl: 7.31, N: 11.52, O: 16.41, S: 13.15. ¹H-NMR (400 MHz, D₂O): δ: 2.01 (m, 2H), 2.22 (m, 2H), 2.44 (s, 3H), 2.85 (s, 6H), 3.18 (m, 2H), 6.50 (m, 1H), 7.20 (m, 1H), 7.40 (m, 1H), 7.50 (m, 1H), 7.58 (m, 1H), 7.85 (m, 1H).

### Preparation of 6-chloro-4-N,N-dimethylaminobutyryloxy-2-methyl-N-2-pyridinyl-2H-thieno[2,3-e]-1,2-thiazine-3-carboxamide 1,1-dioxide.HCl

36 g (0.1 mol) of 6-chloro-4-hydroxy-2-methyl-N-2-pyridinyl-2H-thieno[2,3-e] - 1,2-thiazine-3-carboxamide 1,1-dioxide.HCl was dissolved in 200 ml of acetone and 200 ml of 10% NaHCO₃. 22.3 g (0.12 mol) of dimethylaminobutyryl chloride hydrochloride was added into the mixture and the mixture was stirred for 3 hours at RT. The solvents were evaporated off. 500 ml of ethyl acetate was added into the reaction mixture and the mixture was washed with 10 % NaHCO₃ (1 x 500 ml) and water (3 x 100 ml). The organic solution was dried over anhydrous sodium sulfate. Sodium sulfate was removed by filtration. HCl gas (4 g) is bubbled into the solution. The solid product was collected by filtration. After drying, it yielded 42 g of the hygroscopic desired product (80.5%). Solubility in water: 250 mg/ml; Elementary analysis: C₁₉H₂₂ Cl₂N₄O₅S₂; MW: 521.44. Calculated % C: 43.76, H: 4.25, Cl: 13.60, N: 10.74, O: 15.34, S: 12.30; Found % C: 43.72, H: 4.27, Cl: 13.67, N: 10.70; O: 15.37, S: 12.27. ¹ H-NMR (400 MHz, D₂O): δ: 2.02 (m, 2H), 2.21 (m, 2H), 2.47 (s, 3H), 2.86 (s, 6H), 3.18 (m, 2H), 6.60-6.70 (m, 2H), 7.10 (s, 1H), 7.44 (m, 1 H), 8.10 (m, 1H).

### Preparation of 4-N,N-dimethylaminobutyryloxy-2-methyl-N-2-pyridinyl-2H-thieno[2,3.e]-1,2-thiazi ne-3-carboxamide 1,1-dioxide-HCl.

32.5 g (0.1 mol) of 4-hydroxy-2-methyl-N-2-pyridinyl-2H-thieno[2,3-e] - 1,2-thiazine-3-carboxamide 1,1-dioxide and 16 g (0.1 mol) of diethylaminobutyric acid were dissolved in 300 ml of dichloromethylene. The mixture is cooled to 0 °C with ice bath. 20.6 g (0.1 mol) of N, N'-Dicyclohexylcarbodiimide was added into the reaction mixture. The mixture was stirred for 1 hour at 0 °C and 2 hours at RT. The solid is removed by filtration. The dichloromethylene solution was washed with 5% NaHCO₃ (2 x 100 ml) and water (3 x 100 ml). The organic solution was dried over anhydrous sodium sulfate. Sodium sulfate was removed by filtration. HCl gas (4 g) is bubbled into the solution. The solid product was collected by filtration. After drying, it yielded 39 g of the hygroscopic desired product (80.1 %). Solubility in water: 250 mg/ml; Elementary analysis: C₁₉H₂₃ClN₄O₅S₂; MW: 486.99. Calculated % C: 46.86, H: 4.76, Cl: 7.28, N: 11.50, O: 16.43, S: 13.17; Found % C: 46.82, H: 4.77, Cl: 7.30, N: 11.47; O: 16.47, S: 13.15. ¹H-NMR (400 MHz, D₂O): δ: 2.02 (m, 2H), 2.21 (m, 2H), 2.47 (s, 3H), 2.86 (s, 6H), 3.18 (m, 2H), 6.61-6.70 (m, 2H), 7.30 (d, 1H), 7.45 (m, 1H), 7.60 (d, 1H), 8.11 (m, 1H).

### Preparation of 4-N,N-dimethylaminobutyryloxy-2-methyl-N-[5-Methyl-3-isoxolyl-2H-1,2-benzothiazine-3-carboxamide 1,1-dioxide)-HCl

32.5g (0.1 mol) of 4- hydroxy - 2-methyl-N-[5-Methyl-3-isoxolyl-2H-1,2-benzothiazine-3-carboxamide 1,1-dioxide] .HCl and 16 g (0.1 mol) of diethylaminobutyric acid were dissolved in 300 ml of dichloromethylene. The mixture is cooled to 0 °C with ice bath. 20.6 g (0.1 mol) of N, N'-Dicyclohexylcarbodiimide was added into the reaction mixture. The mixture was stirred for 1 hour at 0 °C and 2 hours at RT. The solid is removed by filtration. The dichloromethylene solution was washed with 5% NaHCO₃ (2 x 100 ml) and water (3 x 100 ml). The organic solution was dried over anhydrous sodium sulfate. Sodium sulfate was removed by filtration. HCl gas (4 g) is bubbled into the solution. The solid product was collected by filtration. After drying, it yielded 37 g of the hygroscopic desired product (78.7%). Solubility in water: 250 mg/ml; Elementary analysis: C₁₉H₂₃ ClN₄O₆S; MW: 470.93. Calculated % C: 48.46, H: 4.92, Cl: 7.53, N: 11.90, O: 20.38, S: 6.81; Found % C: 48.43, H: 4.94, Cl: 7.57, N: 11.86, O: 20.41, S: 6.79. ¹H-NMR (400 MHz, D₂O): δ: 2.01 (m, 2H), 2.22 (m, 2H), 2.44 (s, 3H), 2.85 (s, 6H), 3.18 (m, 2H), 6.40 (m, 1H), 7.20 (m, 1H), 7.40 (m, 1H), 7.52 (m, 1H), 7.58 (m, 1H), 7.85 (m, 1H).

### Industrial Applicability

The pro-drugs of the general formula (1) 'Structure 1' are superior to oxicams and related compounds. They can be used medicinally in treating any oxicams and related compounds-treatable conditions in humans or animals. They may be used for the relief of signs and symptoms of rheumatoid arthritis and osteoarthritis, the reduction of fever, and the treatment of dysmenorrhea. Due to their very high membrane penetration rate, these pro-drugs can be used in treating asthma by inhalation to a host. They can be used for treating breast cancer, colorestal cancer, pancreatic cancer, skin cancer, and any other cancers and for treating psoriasis, acne, sunburn or other skin disorders due to their anti-inflammatory properties.

## Claims

1. A compound having a general formula of Structure 1 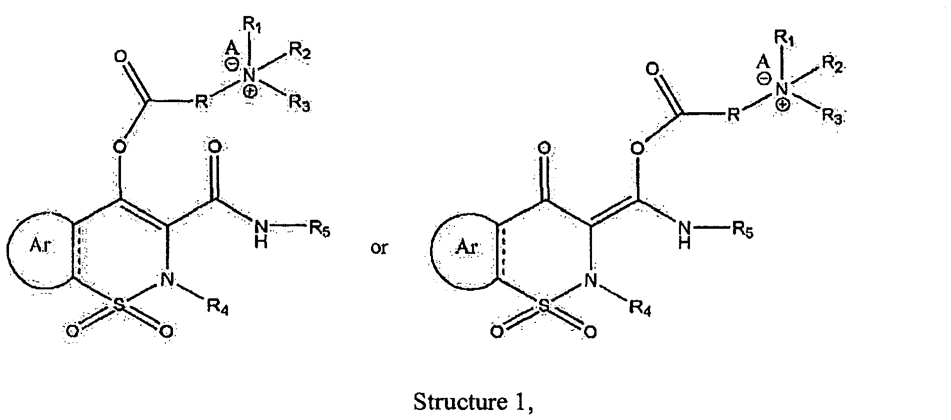 wherein
R is a branched or straight chain, -(CH₂)ₙ-, wherein n=0, 1, 2, 3, 4, 5, 6, 7, 8, 9, or 10;
R₁ is selected from the group consisting of H, alkyl residues having 1 to 12 carbon atoms, alkyloxy residues having 1 to 12 carbon atoms, alkenyl residues having up to 12 carbon atoms, alkynyl residues having up to 12 carbon atoms, aryl residues, and heteroaryl residues;
R₂ is selected from the group consisting of H, alkyl residues having 1 to 12 carbon atoms, alkyloxy residues having 1 to 12 carbon atoms, alkenyl residues having up to 12 carbon atoms, alkynyl residues having up to 12 carbon atoms, aryl residues, and heteroaryl residues;
R₃ is H;
R₄ is selected from the group consisting of H, CH₃, C₂H₅, CF₃, and C₂F₅;
A⁻ is a negative ion;
R₅ represents an aryl or heteroaryl system selected from the group consisting of 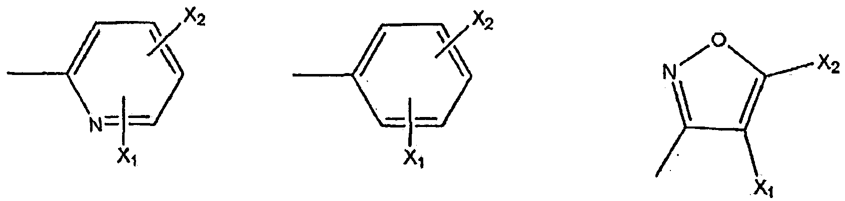 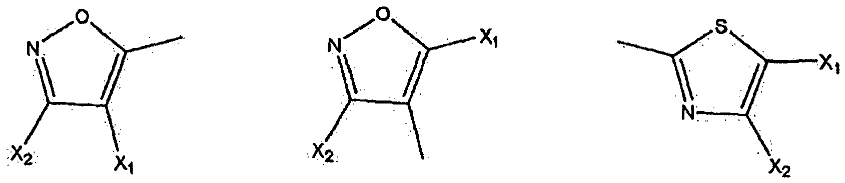 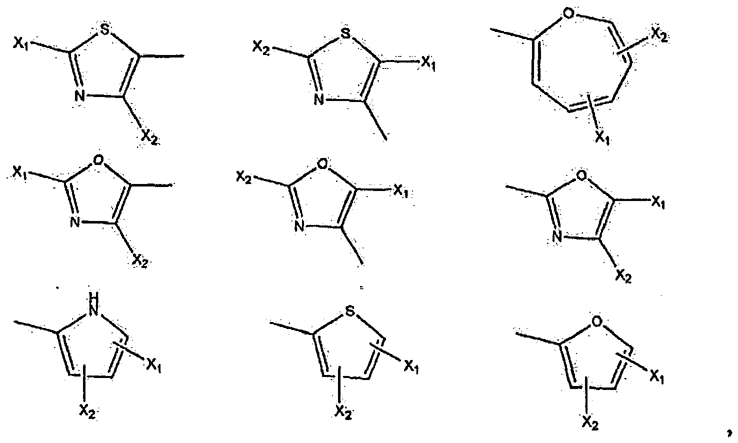 wherein
X₁ and X₂ are independently selected from the group consisting of H, F, Cl, Br, I, CF₃, C₂F₅, SO₂CF₃, SO₂CH₃, NO₂, CN, alkyl residues having 1 to 8 carbon atoms, alkyloxy residues having 1 to 8 carbon atoms, alkenyl residues having up to 8 carbon atoms, and alkynyl residues having up to 8 carbon atoms. 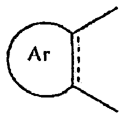 represents an aryl or heteroaryl system which is selected from the group consisting of: 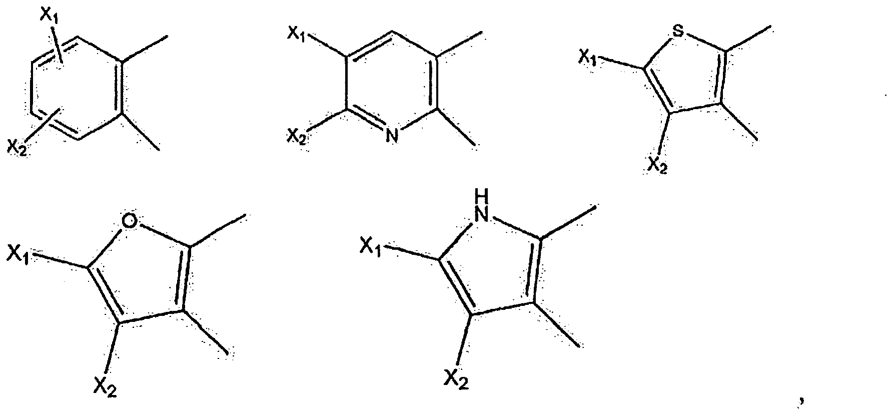 wherein
X₁ and X₂ are independently selected from the group consisting of H, F, Cl, Br, I, CF₃, C₂F₅, SO₂CF₃, SO₂CH₃, NO₂, alkyl residues having 1 to 8 carbon atoms, alkyloxy residues having 1 to 8 carbon atoms, alkenyl residues having up to 8 carbon atoms, and alkynyl residues having up to 8 carbon atoms.

2. Process for the preparation of compounds according to claim 1, wherein metal salts, organic base salts, or immobilized base salts of oxicams and related compounds are reacted with compounds having a general formula of Structure 3 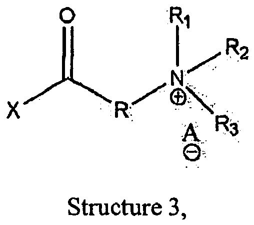 wherein
R is a branched or straight chain, -(CH₂)ₙ -, wherein n=0,1, 2, 3,4, 5, 6, 7, 8, 9, or 10;
R₁ is selected from the group consisting of H, alkyl residues having 1 to 12 carbon atoms, alkyloxy residues having 1 to 12 carbon atoms, alkenyl residues having up to 12 carbon atoms, alkynyl residues having up to 12 carbon atoms, aryl residues, heteroaryl residues;
R₂ is selected from the group consisting of H, alkyl residues having 1 to 12 carbon atoms, alkyloxy residues having 1 to 12 carbon atoms, alkenyl residues having up to 12 carbon atoms, alkynyl residues having up to 12 carbon atoms, aryl residues, and heteroaryl residues;
R₃ is H;
X is halogen or p-toluenesulphonyl;
A⁻ is a negative ion.

3. A compound according to claim 1 or a composition comprising at least one compound according to claim 1, as an active ingredient, for use in the treatment of a oxicam-treatable condition in humans or animals, wherein the condition is selected from the group consisting of pain from a toothache, headache, arthritis and inflammatory pain, fever, cancer, dysmenorrhea, radiation-induced vomiting, diabetic neuropathy and acute migraine headache, hemophilic arthropathy, bone loss, and sunburn, wherein the compound or composition is administered orally or transdermally.

4. A compound according to claim 1 or a composition comprising at least one compound according to claim 1, as an active ingredient, for use in the treatment of a oxicam-treatable condition in humans or animals, wherein the condition is selected from the group consisting of rheumatoid arthritis, osteoarthritis, fever, dysmenorrheal, breast cancer, colorestal cancer, lung cancer, pancreatic cancer, skin cancer, cancer, psoriasis, acne, sunburn, skin disorders, wherein the compound or composition is in the form of a solution, spray, lotion, ointment, emulsion, or gel and administered transdermally.

5. A compound according to claim 1 or a composition comprising at least one compound according to claim 1, as an active ingredient, for use in the treatment of pain in humans or animals, wherein a therapeutically effective amount is topically administered to the inflamed area.

6. The compound for use according to claim 5, wherein the pain is selected from the group consisting of headache, toothache, muscle pain, pain from arthritis, and inflammatory pain.

7. A compound according to claim 1 or a composition comprising at least one compound according to claim 1, as an active ingredient, for use in the treatment of a condition selected from the group consisting of psoriasis, acne, sunburn, and skin disorders, wherein the compound or composition is in the form of a solution, spray, lotion, ointment, emulsion, or gel and administrated transdermally.

8. A compound according to claim 1 or a composition comprising at least one compound according to claim 1, as an active ingredient, for use in the treatment of asthma, wherein the compound or composition is a) administered via inhalation to a host, or b) administered via spraying into the mouth or nose of the host or via spraying to other parts of the body.

9. A compound according to claim 1 or a composition comprising at least one compound according to claim 1, as an active ingredient, for use in the treatment of a condition in humans or animals, wherein the condition is selected from the group consisting of eye inflammatory diseases, ocular pain after corneal surgery, glaucoma, ear inflammatory conditions, ear painful conditions, and otitis.

10. A compound according to claim 1 or a composition comprising at least one compound according to claim 1, as an active ingredient, for use in the treatment of cancer, breast cancer, colorestal cancer, lung cancer, pancreatic cancer, and skin cancer.

11. A transdermal therapeutic application system comprising a compound according to claim 1 or a composition comprising at least one compound according to claim 1, as an active ingredient, for use in the treatment of any NSAIAs-treatable conditions in humans or animals, wherein the conditions are selected from the group consisting of psoriasis, acne, sunburn, skin disorders, cancer, skin cancer, lung cancer, breast cancer, colorestal cancer, pancreatic cancers, pain toothache, headache, arthritis and other inflammatory pain, fever, dysmenorrhea, radiation-induced vomiting, diabetic neuropathy, acute migraine headache, hemophilic arthropathy, and bone loss.

12. The transdermal therapeutic application system for use according to claim 11, **characterized in that** the system is a bandage or a patch comprising one active substance-containing matrix layer and an impermeable backing layer.

13. The transdermal therapeutic application system for use according to claim 11 or 12, **characterized by** having an active substance reservoir, which has a permeable bottom facing the skin.

14. The transdermal therapeutic application system for use according to one of claims 11-13, **characterized by** a controlling means controlling the rate of release, enabling the oxicams to reach constantly optimal therapeutic blood levels to increase effectiveness and reduce the side effects of oxicams.

## Patentansprüche

1. Eine Verbindung mit der allgemeinen Formel der Struktur 1 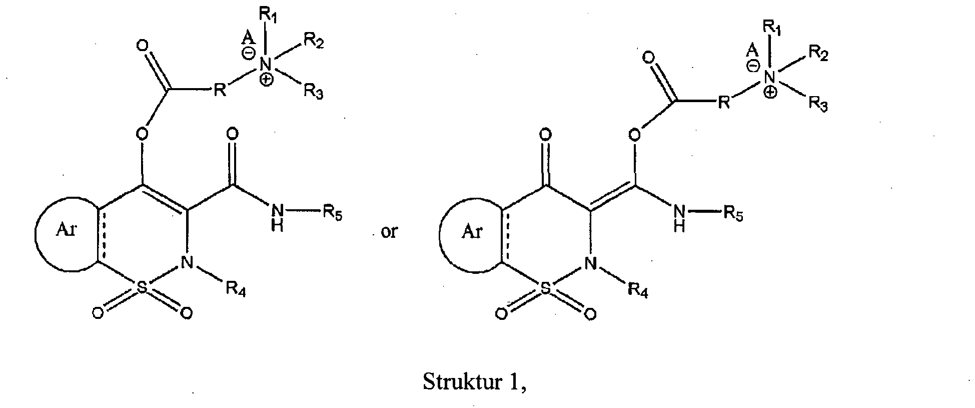 wobei
R ausgewählt ist aus einer verzweigten oder geraden Kette, -(CH₂)ₙ-, wobei n=0, 1, 2, 3, 4, 5, 6, 7, 8, 9 oder 10 ist;
R₁ ausgewählt ist aus der Gruppe bestehend aus H, Alkylreste mit 1 bis 12 Kohlenstoffatomen, Alkyloxyreste mit 1 bis 12 Kohlenstoffatomen, Alkenylreste mit bis zu 12 Kohlenstoffatomen, Alkynylreste mit bis zu 12 Kohlenstoffatomen, Arylreste und Heteroarylreste;
R₂ ausgewählt ist aus der Gruppe bestehend aus H, Alkylreste mit 1 bis 12 Kohlenstoffatomen, Alkyloxyreste mit 1 bis 12 Kohlenstoffatomen, Alkenylreste mit bis zu 12 Kohlenstoffatomen, Alkynylreste mit bis zu 12 Kohlenstoffatomen, Arylreste und Heteroarylreste;
R₃ H ist;
R₄ ausgewählt ist aus der Gruppe bestehend aus H, CH₃, C₂H₅, CF₃, und C₂F₅;
A⁻ ein negatives Ion ist;
R₅ ein Aryl oder Heteroarylsystem darstellt, ausgewählt aus der Gruppe bestehend aus 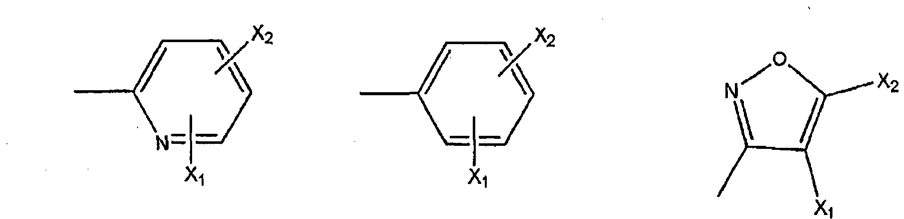 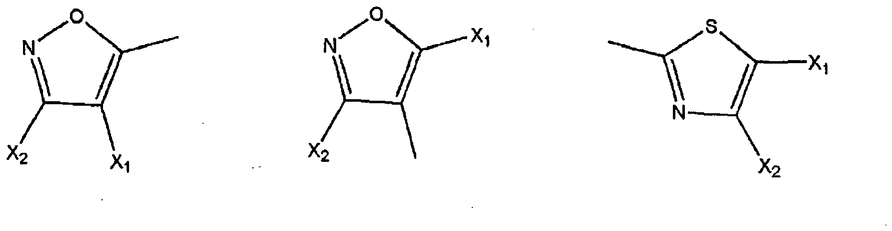 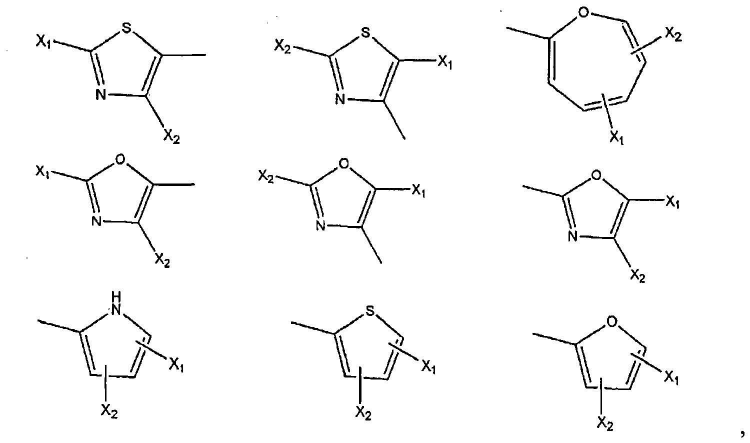 wobei
X₁ und X₂ unabhängig ausgewählt sind aus der Gruppe bestehend aus H, F, Cl, Br, I, CF₃, C₂F₅, SO₂CF₃, SO₂CH₃, NO₂, CN, Alkylresten mit 1 bis 8 Kohlenstoffatomen, Alkyloxyresten mit 1 bis 8 Kohlenstoffatomen, Alkenylresten mit bis zu 8 Kohlenstoffatomen und Alkinylresten mit bis zu 8 Kohlenstoffatomen. 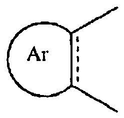 ein Aryl- oder Heteroarylsystem darstellt, welches ausgewählt ist aus der Gruppe bestehend aus 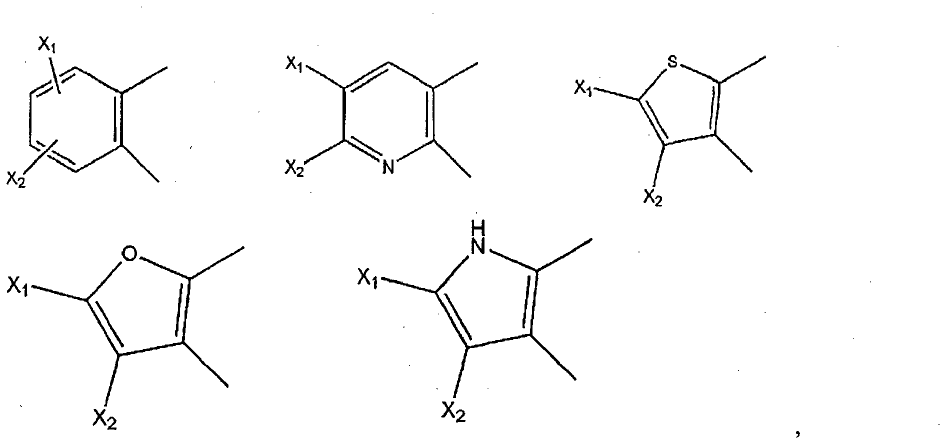 wobei
X₁ und X₂ unabhängig ausgewählt sind aus der Gruppe bestehend aus H, F, Cl, Br, I, CF₃, C₂F₅, SO₂CF₃, SO₂CH₃, NO₂, Alkylresten mit 1 bis 8 Kohlenstoffatomen, Alkyloxyresten mit 1 bis 8 Kohlenstoffatomen, Alkenylresten mit bis zu 8 Kohlenstoffatomen und Alkinylresten mit bis zu 8 Kohlenstoffatomen.

2. Verfahren zur Herstellung von Verbindungen nach Anspruch 1, wobei Metallsalze, organische Basensalze oder immobilisierte Basensalze der Oxicame und verwandter Verbindungen mit Verbindungen der allgemeinen Formel der Struktur 3 zur Reaktion gebracht werden, 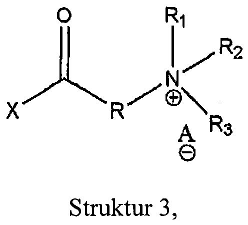 wobei
R eine verzweigte oder gerade Kette, -(CH₂)ₙ-, wobei n=0,1, 2, 3,4, 5, 6, 7, 8, 9 oder 10 ist;
R₁ ausgewählt ist aus der Gruppe bestehend aus H, Alkylresten mit 1 bis 12 Kohlenstoffatomen, Alkyloxyresten mit 1 bis 12 Kohlenstoffatomen, Alkenylresten mit bis zu 12 Kohlenstoffatomen, Alkinylresten mit bis zu 12 Kohlenstoffatomen, Arylreste, Heteroarylreste;
R₂ ausgewählt ist aus der Gruppe bestehend aus H, Alkylresten mit 1 bis 12 Kohlenstoffatomen, Alkyloxyresten mit 1 bis 12 Kohlenstoffatomen, Alkenylreste mit bis zu 12 Kohlenstoffatomen, Alkynylresten mit bis zu 12 Kohlenstoffatomen, Arylresten, Heteroarylresten;
R₃ H ist;
X ein Halogen oder p-Toluolsulfonyl ist;
A⁻ ein negatives Ion ist.

3. Eine Verbindung nach Anspruch 1 oder eine Zusammensetzung, die mindestens aus eine Verbindung nach Anspruch 1 als einen aktiven Bestandteil umfasst, zur Verwendung in der Behandlung eines Oxicam-behandelbaren Leidens in Menschen oder Tieren, wobei das Leiden ausgewählt ist aus der Gruppe bestehend aus Schmerzen durch einen Zahnschmerz, Kopfschmerz, Arthritis- und Entzündungsschmerz, Fieber, Krebs, Dysmenorrhoe, durch Strahlung verursachtes Erbrechen, diabetische Neuropathie und akuten Migränekopfschmerz, hämophile Arthropathie, Knochenschwund und Sonnenbrand, wobei die Verbindung oder die Zusammensetzung oral oder transdermal verabreicht wird.

4. Eine Verbindung nach Anspruch 1 oder eine Zusammensetzung, die mindestens eine Verbindung nach Anspruch 1 als einen aktiven Bestandteil umfasst, zur Verwendung in der Behandlung eines Oxicam-behandelbaren Leidens in Menschen oder Tieren, wobei das Leiden ausgewählt ist aus der Gruppe bestehend aus rheumatoider Arthritis, Osteoarthritis, Fieber, Dysmenorrhoe, Brustkrebs, Darmkrebs, Lungenkrebs, Bauchspeicheldrüsenkrebs, Hautkrebs, Krebs, Psoriasis, Akne, Sonnenbrand, Hauterkrankungen, wobei die Verbindung oder die Zusammensetzung in Form einer Lösung, Spray, Lotion, Salbe, Emulsion oder Gel ist und transdermal verabreicht wird.

5. Verbindung nach Anspruch 1 oder eine Zusammensetzung, die mindestens eine Verbindung nach Anspruch 1 als einen aktiven Bestandteil umfasst, zur Verwendung in der Behandlung von Schmerzen in Menschen oder Tieren, wobei eine therapeutisch effektive Menge dem entzündeten Bereich topisch verabreicht wird.

6. Verbindung nach Anspruch 5, wobei der Schmerz ausgewählt ist aus der Gruppe bestehend aus Kopfschmerzen, Zahnschmerzen, Muskelschmerzen, Schmerzen durch Arthritis und Entzündungsschmerzen.

7. Eine Verbindung nach Anspruch 1 oder eine Zusammensetzung, die mindestens eine Verbindung nach Anspruch 1 als einen aktiven Bestandteil umfasst, zur Verwendung in der Behandlung eines Leidens, ausgewählt aus der Gruppe bestehend aus Psoriasis, Akne, Sonnenbrand und Hauterkrankungen, wobei die Verbindung oder die Zusammensetzung in Form einer Lösung, Spray, Lotion, Salbe, Emulsion oder Gel ist und transdermal verabreicht wird.

8. Eine Verbindung nach Anspruch 1 oder eine Zusammensetzung, die mindestens eine Verbindung nach Anspruch 1 als einen aktiven Bestandteil umfasst, zur Verwendung in der Behandlung von Asthma, wobei die Verbindung oder das Zusammensetzung a) dem Wirt durch Inhalation verabreicht wird oder b) durch Sprühen in den Mund oder in die Nase des Wirts verabreicht wird oder durch das Besprühen anderer Körperteile.

9. Eine Verbindung nach Anspruch 1 oder eine Zusammensetzung, die mindestens eine Verbindung nach Anspruch 1 als einen aktiven Bestandteil umfasst, zur Verwendung in der Behandlung von Leiden in Menschen oder Tieren, wobei das Leiden ausgewählt ist aus der Gruppe bestehend aus Augenentzündungserkrankungen, Augenschmerzen nach einer Hornhautoperation, Glaukom, Ohrentzündungsleiden, schmerzhafte Ohrenleiden und Otitis.

10. Eine Verbindung nach Anspruch 1 oder eine Zusammensetzung, die mindestens eine Verbindung nach Anspruch 1 als einen aktiven Bestandteil umfasst, zur Verwendung in der Behandlung von Krebs, Brustkrebs, Darmkrebs, Lungenkrebs, Bauchspeicheldrüsenkrebs und Hautkrebs.

11. Ein transdermales, therapeutisches Applikationssystem, umfassend eine Verbindung nach Anspruch 1 oder eine Zusammensetzung, die mindestens eine Verbindung nach Anspruch 1 als einen aktiven Bestandteil umfasst, zur Verwendung in einer Behandlung irgendeines NSAIAs-behandelbaren Leidens in Menschen oder Tieren, wobei die Leiden ausgewählt sind aus der Gruppe bestehend aus Psoriasis, Akne, Sonnenbrand, Hauterkrankungen, Krebs, Hautkrebs, Lungenkrebs, Brustkrebs, Darmkrebs, Bauchspeicheldrüsenkrebs, Zahnschmerzen, Kopfschmerzen, Arthritis und anderen Entzündungsschmerzen, Fieber, Dysmenorrhoe, durch Strahlung verursachtes Erbrechen, diabetische Neuropathie, akute Migränekopfschmerzen, hämophile Arthropathie und Knochenschwund.

12. Das transdermale, therapeutische Applikationssystem zur Verwendung nach Anspruch 11, **dadurch gekennzeichnet, dass** das System eine Bandage oder ein Pflaster ist, umfassend eine Matrixschicht, die eine aktive Substanz beinhaltet, und eine impermeable Unterstützungsschicht.

13. Das transdermale, therapeutische Applikationssystem zur Verwendung nach Anspruch 11 oder 12, **dadurch gekennzeichnet, dass** das System ein Reservoir einer aktiven Substanz aufweist, das einen permeablen Boden aufweist, der der Haut zugewendet ist.

14. Das transdermale therapeutische Applikationssystem zur Verwendung nach einem der Ansprüche 11 bis 13, **gekennzeichnet durch** ein Kontrollmittel, das die Rate der Abgabe kontrolliert, und das das Oxicam befähigt, konstant einen optimalen therapeutischen Blutspiegel zu erreichen, um die Effektivität zu erhöhen und die Nebenwirkungen des Oxicams zu verringern.

## Revendications

1. Composé ayant une formule générale de structure 1 : 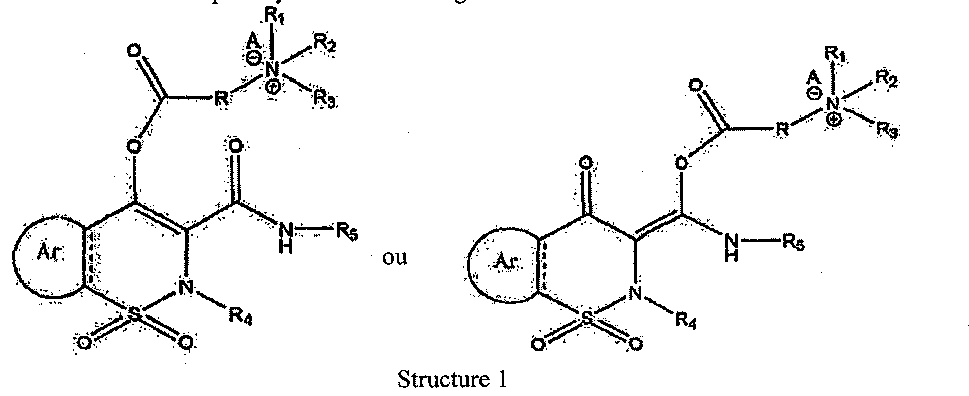 où
R est une chaîne linéaire ou ramifiée, -(CH₂)ₙ-, où n = 0, 1, 2, 3, 4, 5, 6, 7, 8, 9 ou 10 ;
R₁ est choisi dans le groupe constitué par H, les résidus alkyle ayant de 1 à 12 atomes de carbone, les résidus alkyloxy ayant de 1 à 12 atomes de carbone, les résidus alcényle ayant jusqu'à 12 atomes de carbone, les résidus alcynyle ayant jusqu'à 12 atomes de carbone, les résidus aryle, et les résidus hétéroaryle ;
R₂ est choisi dans le groupe constitué par H, les résidus alkyle ayant de 1 à 12 atomes de carbone, les résidus alkyloxy ayant de 1 à 12 atomes de carbone, les résidus alcényle ayant jusqu'à 12 atomes de carbone, les résidus alcynyle ayant jusqu'à 12 atomes de carbone, les résidus aryle, et les résidus hétéroaryle ;
R₃ est H ;
R₄ est choisi dans le groupe constitué par H, CH₃, C₂H₅, CF₃ et C₂F₅ ;
A⁻ est un ion négatif ;
R₅ représente un système aryle ou hétéroaryle choisi dans le groupe constitué par 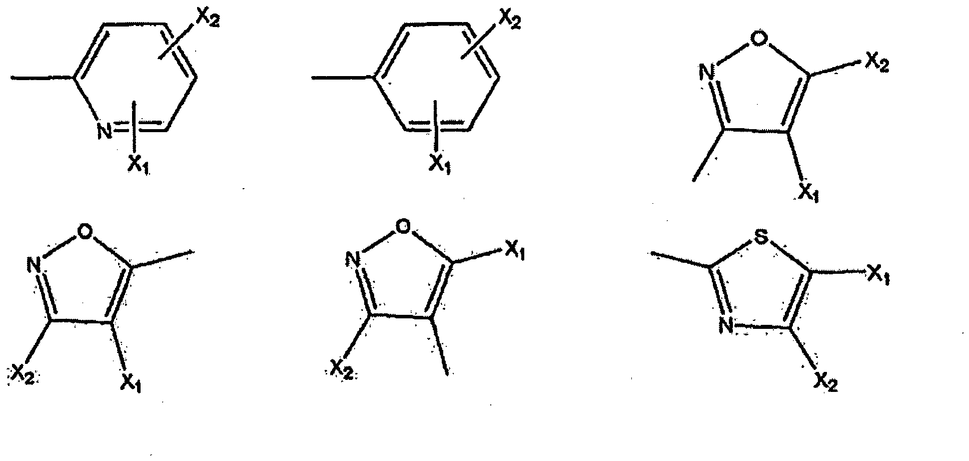 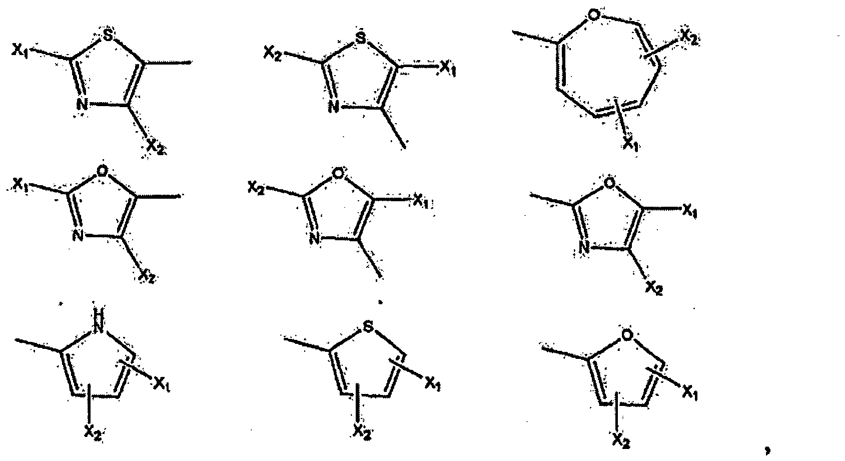 où
X₁ et X₂ sont indépendamment choisis dans le groupe constitué par H, F, Cl, Br, I, CF₃, C₂F₅, SO₂CF₃, SO₂CH₃, NO₂, CN, les résidus alkyle ayant de 1 à 8 atomes de carbone, les résidus alkyloxy ayant de 1 à 8 atomes de carbone, les résidus alcényle ayant jusqu'à 8 atomes de carbone, et les résidus alcynyle ayant jusqu'à 8 atomes de carbone : 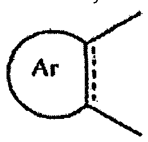 représente un système aryle ou hétéroaryle qui est choisi dans le groupe constitué par : 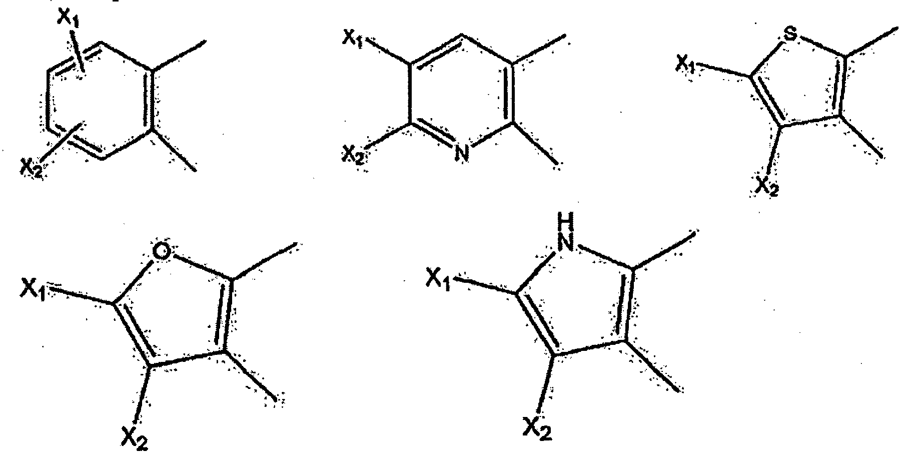 où
X₁ et X₂ sont indépendamment choisis dans le groupe constitué par H, F, Cl, Br, I, CF₃, C₂F₅, SO₂CF₃, SO₂CH₃, NO₂, les résidus alkyle ayant de 1 à 8 atomes de carbone, les résidus alkyloxy ayant de 1 à 8 atomes de carbone, les résidus alcényle ayant jusqu'à 8 atomes de carbone, et les résidus alcynyle ayant jusqu'à 8 atomes de carbone.

2. Procédé de préparation de composés selon la revendication 1, dans lequel des sels métalliques, des sels de base organique, ou des sels de base immobilisée d'oxicams, et des composés apparentés, sont mis à réagir avec des composés ayant une formule générale de structure 3 : 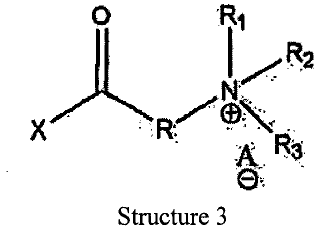 où
R est une chaîne linéaire ou ramifiée, -(CH₂)ₙ-, où n = 0, 1, 2, 3, 4, 5, 6, 7, 8, 9 ou 10 ;
R₁ est choisi dans le groupe constitué par H, les résidus alkyle ayant de 1 à 12 atomes de carbone, les résidus alkyloxy ayant de 1 à 12 atomes de carbone, les résidus alcényle ayant jusqu'à 12 atomes de carbone, les résidus alcynyle ayant jusqu'à 12 atomes de carbone, les résidus aryle, et les résidus hétéroaryle ;
R₂ est choisi dans le groupe constitué par H, les résidus alkyle ayant de 1 à 12 atomes de carbone, les résidus alkyloxy ayant de 1 à 12 atomes de carbone, les résidus alcényle ayant jusqu'à 12 atomes de carbone, les résidus alcynyle ayant jusqu'à 12 atomes de carbone, les résidus aryle, et les résidus hétéroaryle ;
R₃ est H ;
X est un halogène ou p-toluènesulfonyle ;
A⁻ est un ion négatif.

3. Composé selon la revendication 1 ou composition comprenant au moins un composé selon la revendication 1, à titre d'ingrédient actif, pour une utilisation dans le traitement d'une affection pouvant être traitée par un oxicam chez les humains ou les animaux, dans lequel l'affection est choisie dans le groupe constitué par une douleur due à une rage de dents, une céphalée, l'arthrite et une douleur inflammatoire, une fièvre, un cancer, une dysménorrhée, des vomissements induits par des rayonnements, une neuropathie diabétique et une céphalée de type migraine aiguë, une arthropathie hémophile, une perte osseuse, et un érythème solaire, lequel composé ou composition est administré par voie orale ou transdermique.

4. Composé selon la revendication 1 ou composition comprenant au moins un composé selon la revendication 1, à titre d'ingrédient actif, pour une utilisation dans le traitement d'une affection pouvant être traitée par un oxicam chez les humains ou les animaux, dans lequel l'affection est choisie dans le groupe constitué par la polyarthrite rhumatoïde, l'arthrose, une fièvre, une dysménorrhée, un cancer du sein, un cancer colorectal, un cancer du poumon, un cancer du pancréas, un cancer de la peau, un cancer, le psoriasis, l'acné, un érythème solaire, des troubles cutanés, lequel composé ou composition est sous la forme d'une solution, d'un spray, d'une lotion, d'une pommade, d'une émulsion ou d'un gel et est administré par voie transdermique.

5. Composé selon la revendication 1 ou composition comprenant au moins un composé selon la revendication 1, à titre d'ingrédient actif, pour une utilisation dans le traitement d'une douleur chez les humains ou les animaux, dans lequel une quantité thérapeutiquement efficace est administrée par voie topique à la zone enflammée.

6. Composé pour une utilisation selon la revendication 5, dans lequel la douleur est choisie dans le groupe constitué par une céphalée, une rage de dents, une douleur musculaire, une douleur liée à l'arthrite, et une douleur inflammatoire.

7. Composé selon la revendication 1 ou composition comprenant au moins un composé selon la revendication 1, à titre d'ingrédient actif, pour une utilisation dans le traitement d'une affection choisie dans le groupe constitué par le psoriasis, l'acné, un érythème solaire et des troubles cutanés, lequel composé ou composition est sous la forme d'une solution, d'un spray, d'une lotion, d'une pommade, d'une émulsion ou d'un gel et est administré par voie transdermique.

8. Composé selon la revendication 1 ou composition comprenant au moins un composé selon la revendication 1, à titre d'ingrédient actif, pour une utilisation dans le traitement de l'asthme, lequel composé ou composition est a) administré par inhalation à un hôte ou b) administré par pulvérisation dans la bouche ou le nez de l'hôte ou par pulvérisation à d'autres parties du corps.

9. Composé selon la revendication 1 ou composition comprenant au moins un composé selon la revendication 1, à titre d'ingrédient actif, pour une utilisation dans le traitement d'une affection chez les humains ou les animaux, dans lequel l'affection est choisie dans le groupe constitué par les maladies inflammatoires oculaires, les douleurs oculaires après une opération de la cornée, un glaucome, les affections inflammatoires auriculaires, les affections auriculaires douloureuses, et l'otite.

10. Composé selon la revendication 1 ou composition comprenant au moins un composé selon la revendication 1, à titre d'ingrédient actif, pour une utilisation dans le traitement d'un cancer, d'un cancer du sein, d'un cancer colorectal, d'un cancer du poumon, d'un cancer du pancréas, et d'un cancer de la peau.

11. Système d'application thérapeutique transdermique comprenant un composé selon la revendication 1 ou une composition au moins un composé selon la revendication 1, à titre d'ingrédient actif, pour une utilisation dans le traitement de quelconques affections pouvant être traitées par des agents AINS chez les humains ou les animaux, dans lequel les affections sont choisies dans le groupe constitué par le psoriasis, l'acné, un érythème solaire, des troubles cutanés, un cancer, un cancer de la peau, un cancer du poumon, un cancer du sein, un cancer colorectal, les cancers du pancréas, une rage de dents douloureuse, une céphalée, l'arthrite et une autre douleur inflammatoire, une fièvre, la dysménorrhée, des vomissements induits par des rayonnements, une neuropathie diabétique, une céphalée de type migraine aiguë, une arthropathie hémophile, et une perte osseuse.

12. Système d'application thérapeutique transdermique pour une utilisation selon la revendication 11, **caractérisé en ce que** le système est un bandage ou un timbre comprenant une couche de matrice contenant une substance active et une couche d'envers imperméable.

13. Système d'application thérapeutique transdermique pour une utilisation selon la revendication 11 ou 12, **caractérisé en ce qu'**il a un réservoir de substance active, qui a un fond perméable faisant face à la peau.

14. Système d'application thérapeutique transdermique pour une utilisation selon l'une quelconque des revendications 11 à 13, **caractérisé par** un moyen de contrôle qui contrôle la vitesse de libération, en permettant aux oxicams d'atteindre constamment des niveaux sanguins thérapeutiques optimaux pour accroître l'efficacité et réduire les effets secondaires des oxicams.
